# EUROPÄISCHE PATENTSCHRIFT

(11) **EP 2 066 374 B1**
(45) Veröffentlichungstag und Bekanntmachung des Hinweises auf die Patenterteilung: **06.10.2010**
(21) Anmeldenummer: 07802027.8
(22) Anmeldetag: 31.08.2007
(51) Int. Cl.: A61M 5/315

(54) **ZYLINDERKOLBENEINHEIT MIT MINDESTENS DREI DICHTELEMENTEN**
CYLINDER/PISTON UNIT WITH AT LEAST THREE SEALING ELEMENTS
ENSEMBLE CYLINDRE-PISTON AVEC AU MOINS TROIS ÉLÉMENTS D'ÉTANCHÉITÉ

(30) Priorität: 27.09.2006 DE 102006045959
(43) Veröffentlichungstag der Anmeldung: 10.06.2009
(73) Patentinhaber: LTS LOHMANN Therapie-Systeme AG, 56626 Andernach (DE)
(72) Erfinder: HOFFMANN, Hans-Rainer, 56566 Neuwied (DE); MATUSCH, Rudolf, 35041 Marburg (DE)
(74) Vertreter: Schmidt, Werner
(86) Internationale Anmeldenummer: PCT/EP2007/007609
(87) Internationale Veröffentlichungsnummer: WO 2008/037329

(56) Entgegenhaltungen:
- WO-A-88/05315
- WO-A-99/44659
- GB-A- 1 168 201
- JP-A- 2000 342 688

## Beschreibung

Die Erfindung betrifft eine Zylinderkolbeneinheit mit einem Zylinder und einem darin geführten, steril gummiabgedichteten Kolben, wobei der Zylinder und der Kolben eine zumindest zeitweise wirkstoffbefüllbare Kammer umschließen und der Zylinder an seinem vorderen Ende mindestens ein Austrittselement aufweist.

Bei befüllt gelagerten Arzneimittelkammern oder Spritzen, die u.a. mittels eines Kolbens steril verschlossen sind, ist der mit der Injektionslösung in Kontakt kommende Kolben aus Gummi oder er hat zumindest eine Gummidichtung. Da ein solcher steril dichtender Kolben eine hohe Haft- und Gleitreibung gegenüber dem Glas- oder Kunststoffzylinder aufweist, wird der Kolben nach DIN 13098, Teil 1, beispielsweise mit Polydimethylsiloxan geschmiert. Mit der Injektionslösung wird somit zusätzlich das Schmiermittel verabreicht.

Eine andere Alternative ist das Versiegeln der Arzneimittelkammer bzw. des Spritzenzylinders, siehe DE 10 2005 054 600. In dieser Druckschrift haben die Kolben keine Gummidichtungen. Beispielsweise wird hier die Rückseite einer Arzneimittelkammer mit einer Folie heißversiegelt, oder mit einem Sprühlack steril verschlossen.

Der vorliegenden Erfindung liegt daher die Problemstellung zugrunde, eine vorbefüllbare Zylinderkolbeneinheit zu entwickeln, bei der trotz einer sterilen Kolbenabdichtung nur ein geringer Kraftbedarf notwendig ist, um den Kolben zu beschleunigen und/oder zu bewegen.

Diese Problemstellung wird mit den Merkmalen des Hauptanspruches gelöst. Dazu ist der in einer hinteren Position ruhende Kolben gegenüber dem Zylinder mit einem statischen vorderen und mit einem statischen hinteren Dichtelement steril abgedichtet, wobei beide Dichtelemente in einer Dichtstellung jeweils an der Wandung des Zylinders und jeweils an der Wandung des Kolbens (50) anliegen. Räumlich hinter jedem statischen Dichtelement ist ein das jeweilige Dichtelement aufnehmender Parkraum angeordnet. Bei betätigtem Kolben werden die einzelnen statischen Dichtelemente aus ihrer jeweiligen Dichtstellung heraus in eine im Parkraum gelegene Parkstellung überführt, wobei jedes Dichtelement in der Parkstellung entweder nur die Zylinderwandung oder nur die Kolbenwandung kontaktiert. Zwischen den beiden statischen Dichtelementen ist mindestens ein dynamisches kolbenseitiges Dichtelement angeordnet, das zumindest bei betätigtem Kolben an der Zylinderwandung anliegt.

Mit der Erfindung wird eine z.B. in einer Subkutaninjektionsvorrichtung verwendbare Zylinder-Kolben-Einheit geschaffen, bei der durch die konstruktive Gestaltung der zwischen der Zylinderinnenwandung und der Kolbenaußenkontur liegenden Dichtungen und ihrer Dichtungssitze der Kolben bei seiner Arbeitsbewegung nur einen geringen Reibungswiderstand erzeugt. Zudem weist die Zylinderkolbeneinheit einen nach dem technischen Prinzip der Selbsthilfe selbstabdichtenden Kolben auf, der u.a. aufgrund seiner Dichtmittelgestaltung schmiermittelfrei im Zylinder sitzt.

Bei der beschriebenen Zylinder-Kolben-Einheit wird der Kolben beispielsweise im Vakuumsetzverfahren in die vorbefüllte Medikamentenkammer eingesetzt. Im Vakuum wird dabei der Kolben auf den Flüssigkeitsspiegel in der Bohrung der Medikamentenkammer aufgesetzt. Alle Hohlräume in der Umgebung des Kolbens sind somit vakuumisiert, sofern sie vor dem hinteren, statischen Dichtelement liegen. Letzteres dichtet die Bewegungsfuge der Zylinder-Kolben-Einheit gegen die Umgebung ab.

Wird nun der Kolben zum Austreiben der Injektionslösung beschleunigt, wird das vordere Dichtelement aufgrund des Flüssigkeitsdrucks aus seiner Dichtstellung in eine Parkstellung verlagert. Dabei verliert dieses statische Dichtelement seinen reibenden Kontakt zum Zylinder. Eine reguläre, dynamische Kolbendichtung, die nur einen geringen Gleitreibungswiderstand hat, übernimmt die Abdichtung zwischen Kolben und Zylinder. Das hintere statische Dichtelement haftet beispielsweise aufgrund seiner großen Haftreibung weiterhin am Zylinder, obwohl sich der Kolben schon abwärts bewegt. Die Haftreibung führt zu einem Abstreifen dieses Dichtelementes. Es rutscht in eine Parkstellung, in der es die Kolbenbewegung nicht behindern kann.

Weitere Einzelheiten der Erfindung ergeben sich aus den Unteransprüchen und den nachfolgenden Beschreibungen schematisch dargestellter Ausführungsbeispiele.
- Figur 1:: Zylinderkolbeneinheit mit einem in der oberen Endlage positionierten Kolben;
- Figur 2:: Seitenansicht zu Figur 1;
- Figur 3:: Zylinderkolbeneinheit beim Flüssigkeitsausstoß;
- Figur 4:: wie Figur 3, jedoch mit einem anderen, vorderen Kolbenabschnitt;
- Figur 5:: wie Figur 1, jedoch mit anderen, hinteren Dichtelementorten;
- Figur 6:: wie Figur 5, jedoch beim Flüssigkeitsausstoß;
- Figur 7:: teilgeschnittener betätigter Zylinderkolben mit Querschnitten;
- Figur 8:: Zylinderkolbeneinheit mit vorderer Lippendichtung;
- Figur 9:: Zylinderkolbeneinheit mit langem Kolben.

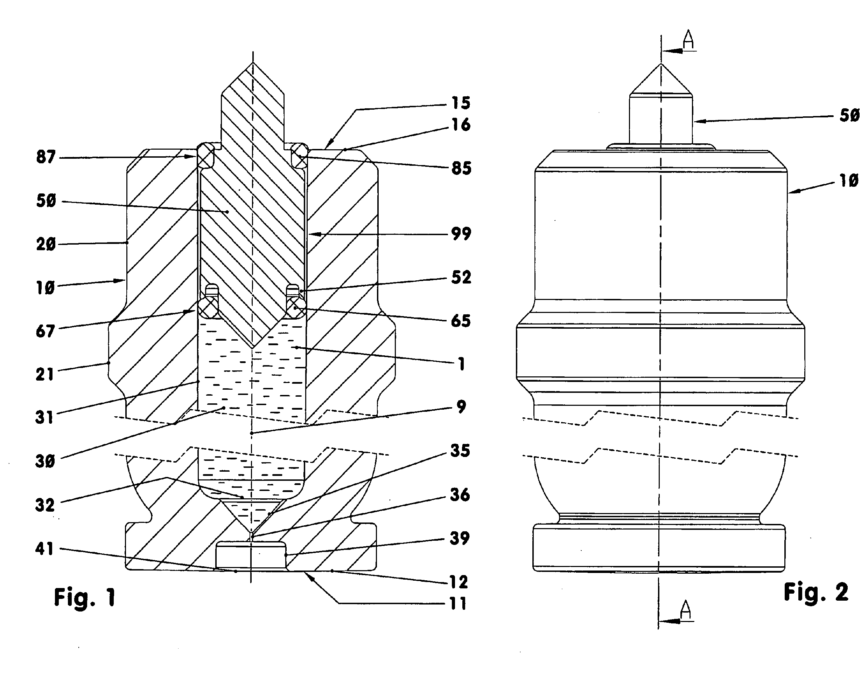

Die Figur 1 zeigt eine Zylinderkolbeneinheit, wie sie beispielsweise in einer nadelfreien Subkutaninjektionsvorrichtung verwendet wird. Sie umfasst einen Zylinder (10) und einen z.B. kolbenstangenfreien Kolben (50) mit einem vorderen (65) und einem hinteren Dichtelement (85) aus Gummi. Beide schließen in einer Kammer (30) ein subkutan zu verabreichendes Präparat (1) oder ein flüssiges Trägermaterial, z.B. destilliertes Wasser oder physiologische Kochsalzlösung, ein. Der Kolben (50) ist in Figur 1 in einer hinteren Position (99) dargestellt. Die Zylinderkolbeneinheit ist beispielsweise für den einmaligen Gebrauch vorgesehen. Sie dient der Verabreichung eines Arzneinennvolumens von z.B. 0,1 bis 2 ml. Ggf. kann auch ein Arzneinennvolumen von 3 ml realisiert werden. Der hier nur beispielsweise ohne integrierte Injektionsnadel ausgeführte Zylinder (10) hält bei einer Verwendung in einer Subkutaninjektionsvorrichtung einer temporären Druckbelastung von mindestens 300 x 10⁵ Pa stand.

Der Zylinder (10) hat in erster Näherung die Form des Spitzenzylinders einer genormten Einmalspritze. Am vorderen Ende (11) befindet sich ein düsenartiges Austrittselement (36), das in der vorderen - beispielsweise planen - Zylinderstirnseite (12) mit einer z.B. kreisrunden Öffnung (41) einer Freistrahlausnehmung (39) endet. Ggf. kann anstelle des düsenartigen Austrittselements eine in den vorliegenden Figuren nicht dargestellte Injektionsnadel sitzen.

Am hinteren Ende des Zylinders (10) befindet sich die hintere Zylinderstirnfläche (16). Sie ist eben und normal zur Zylindermittellinie (9) orientiert.

Zwischen der hinteren (16) und der vorderen Stirnfläche (12) befindet sich eine Außenkontur (20) mit beliebiger Mantelfläche. Die Form der Außenkontur(20) des Zylinders (10) ist in den meisten Fällen unabhängig von der funktionalen Bauteilbezeichnung "Zylinder (10)". Die Außenkontur (20) kann u.a. eine oder mehrere partielle Abflachungen, Flansche, Gewindeabschnitte, Bajonettverschlussteile oder dergleichen aufweisen, um ein Adaptieren an einen Injektor zu ermöglichen und um ggf. ein unbeabsichtigtes seitliches Wegrollen beim Handhaben auf einer ebenen Unterlage zu verhindern.

Die Figuren 1 bis 6 zeigen an der Außenkontur (20) einen ca. mittig angeordneten Adapterflansch (21). Er dient der formsteifen Fixierung an der nicht dargestellten Subkutaninjektionsvorrichtung. Hierbei umgreift ein Bund des Injektorgehäuses oder eine andere Adapterkontur den entsprechenden Flansch (21) des Zylinders (10). Bei einer Injektorvariante mit einem nahezu vollständigen injektorgehäuseseitigen Zylinderumgriff kann ggf. auf einen Adapter verzichtet werden.

Der Außendurchmesser des Adapterflansches (21) ist z.B. mindestens um ein Fünftel der Zylinderwandstärke größer als der Außendurchmesser der benachbarten Außenkontur (20) des Zylinders (10). Auch der Flansch (21) kann seitlich zur Behinderung einer Wegrollbewegung eine oder mehrere Abflachungen aufweisen. Statt der Abflachung sind auch Kerben, Nuten, Sicken oder Riffelungen denkbar.

In Figur 2 ist ein befüllter Zylinder (10) mit eingesetztem steril abdichtenden Kolben (59) in einer Seitenansicht dargestellt.

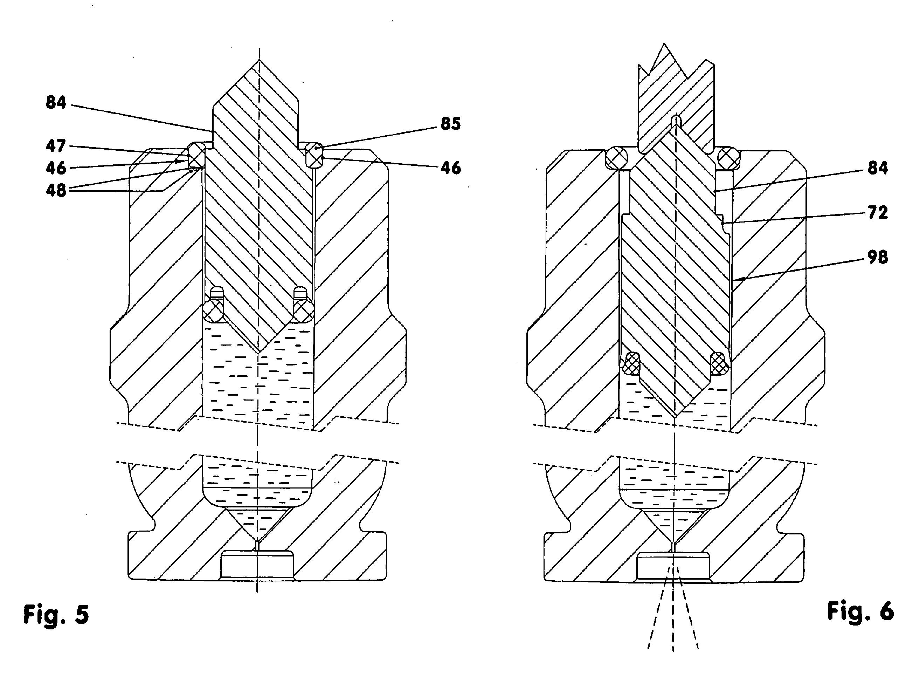

Die Innenkontur des Zylinders (10) umfasst die Zylinderinnenwandung (31), ggf. mit einem Gehäusebund (46), vgl. Figuren 5 und 6, einen Zylinderboden (32), einen Ausströmtrichter (35), eine Düsenbohrung (36) und eine Freistrahlausnehmung (39).

Die z.B. glatte Zylinderinnenwandung (31) verjüngt sich nach den Ausführungsbeispielen linear von hinten nach vorn. Sie erstreckt sich zudem nach den Figuren 1, 3 bis 6 über den gesamten Kolbenhubbereich. Auch sind alle Querschnitte der Zylinderinnenwandung (31) außerhalb des Bereichs des oder der Ausströmtrichter (35) kreisrund. Nur beispielsweise verjüngt sich die Zylinderinnenwandung (31) auf einem Kolbenhub (3) von 18 Millimeter von einem Durchmesser von 7 Millimeter auf 6 Millimeter. Das entspricht einem Verjüngungswinkel von ca. 3,2 Winkelgraden.

Anstelle der dargestellten Spezialfälle können die Querschnitte über den Kolbenhub neben ihrer Fläche auch ihre Form ändern. So könnte die Zylinderinnenwandung an ihrem hinteren Ende z.B. eine ovale Querschnittsform haben, während ein dem vorderen Ende näherliegender Querschnitt eine runde oder polygonartige Form hat. Ferner ist es auch möglich, dass die Querschnittsflächenänderung über den Kolbenhub nicht linear ist. Beispielsweise kann zur Verringerung der Kolbenbremswirkung die Verjüngung erst im letzten Drittel des Ausstoßhubs einsetzen. Der Übergang zwischen Abschnitten unterschiedlicher Querschnitte ist in der Regel stetig gestaltet.

Der Ausströmtrichter (35) verjüngt sich zwischen dem Zylinderboden (32) und der Düsenbohrung (36) z.B. linear. Die Düsenbohrung (36), deren Durchmesser z.B. zwischen 0,1 und 0,4 Millimetern liegt, ist zwei- bis viermal so lang wie ihr Durchmesser. An die Düsenbohrung (36) schließt sich eine zylinderkammerartige Freistrahlausnehmung (39) an. Die Ausnehmung (39) hat einen planen Boden, der zudem normal zur Mittellinie der Düsenbohrung (36) orientiert ist. Ihr Durchmesser entspricht dem achtbis sechzehnfachen des Düsenbohrungsdurchmessers, sofern die Ausnehmungstiefe mindestens doppelt so groß ist wie die Düsenbohrungslänge.

Am hinteren Ende (15) des Zylinders (10) befindet sich im Übergangsbereich der Zylinderinnenwandung (31) und der Stirnfläche(16) der Gehäusebund (46), vgl. Figuren 5 und 6. Der Gehäusebund (46) hat eine radiale (47) und eine axiale Bundfläche (48). Die hinteren Bereiche des Zylinders (10) und des Kolbens (50) sind in den Figuren 5 und 6 - zur Einsparung weiterer Zeichnungen - asymmetrisch dargestellt. Jeweils die rechte Seite der Figur 5 gehört zur rechten Seite der Figur 6. Ebenso passen nur die linken Seiten der Figuren 5 und 6 zusammen.

Die radiale Bundfläche (47) hat auf der jeweils linken Seite einen größeren Durchmesser als auf der rechten Seite. Die Bundflächen (47) sind nur beispielsweise zylindrisch dargestellt. Sie können zur Erhöhung der Dichtwirkung z.B. mit umlaufenden Erhebungen oder anderen Strukturen versehnen sein. Die axialen Dichtflächen (48) sind plan ausgeführt. In Figur 5 ist auch eine gestrichelt dargestellte Dichtfläche (48) gezeigt, die die Form eines Kegelmantelstumpfes hat.

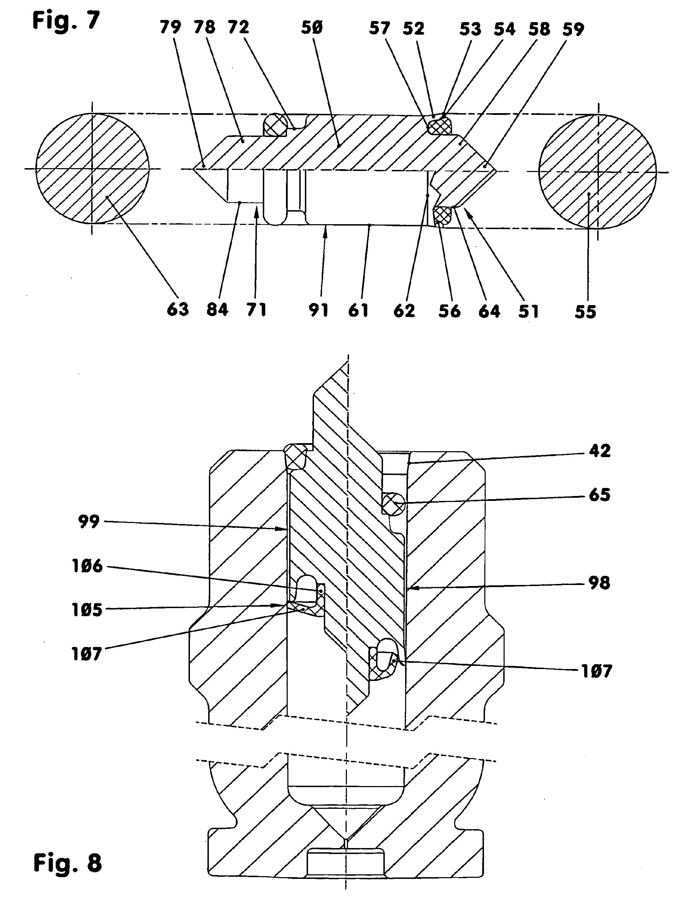

Ggf. kann zwischen der Zylinderinnenwandung (31) und der hinteren Stirnfläche (16) zur einfacheren Montage des Kolbens (10) z.B. eine 5°-Fase (42) vorgesehen sein, vgl. Figur 8.

Als Werkstoff für den Zylinder (10) wird ein transparenter, amorpher Thermoplast, z.B. ein Copolymer auf der Basis von Cycloolefinen und Ethylenen oder α-Olefinen (COC) verwendet. Für Zylinder (10), die unmittelbar vor der Injektion befüllt werden, eignet sich auch Polycarbonat, z.B. Makrolon ^{®}.

Der im Zylinder (10) geführte Kolben (50) muss die Querschnittsänderung der Zylinderinnenwandung durch eine entsprechende Dichtquerschnittsverringerung ausgleichen. Hierbei darf die Wandungsreibung nur unwesentlich zunehmen.

Um dies u.a. zu erreichen, hat der in drei Abschnitte (51, 71, 91) aufteilbare Kolben (50) im vorderen Abschnitt (51) eine Dichtlippe in Form einer Schürze (52), vgl. Figur 7. An den Abschnitt (51) schließen sich nacheinander der mittlere Abschnitt (91) und der hintere Abschnitt (71) an.

Der Mittelabschnitt (91) hat die Form eines Zylinders. Er passt verformungsfrei in den gesamten Hubbereich der Kammer (30). Nach vorn schließt sich mittig ein vorderer Kolbenkern (58) an, der in einer z.B. kegeligen Kolbenspitze (59) endet. Um den Kern (58) herum befindet sich die Schürze (52), wobei die - in den Figuren 1, 2 und 5 bis 7 zylindrisch dargestellte - Kernwandung (64) über die Schürze (52) übersteht. Zwischen der Schürze (52) und dem Kern (58) liegt nach Figur 7 eine Axialringnut (57). Der Durchmesser der Kernwandung (64) ist so gewählt, dass das Dichtelement (65) in der Dichtstellung (67), vgl. Figur 1, mit ausreichender Dichtkraft zwischen der Zylinderinnenwandung (31) und der Kernwandung (64) klemmt.

Die Schürze (52), die sich z.B. über ein Neuntel bis ein Viertel der Kolbenlänge erstreckt, ist ein dünnwandiger, sich im unbelasteten Zustand trichterförmig öffnender Ring. Die vordere Außenkante (53) der Schürze (52) umgibt eine Querschnittsfläche (55), die nach Figur 7 größer ist als eine Querschnittsfläche (63), deren Umfang durch eine - am Fuß der Schürze (52) liegende - gedachte Umrisslinie (62) bestimmt wird. Die Umrisslinie (62) ist in einer Teilansicht des Kolbens (10) in Figur 7 dargestellt. Zwischen der Schürze (52) und der Kernwandung (64) liegt der Parkraum (68).

Auf der Rückseite des Kolbens (50) ist ein hinterer Kolbenkern (78) angeordnet, der ebenfalls in einer kegeligen Kolbenspitze (79) endet. Beide Kolbenspitzen (59, 79) haben jeweils einen Kegelwinkel von z.B. 90 Winkelgraden. Zwischen dem Kolbenkern (78) und dem Mittelabschnitt (91) befindet sich ein Wellenbund (72), dessen Durchmesser gerade so groß ist, dass das Dichtelement (85) in der Dichtstellung (87), vgl. Figur 1, mit genügend Dichtkraft zwischen der Zylinderinnenwandung (31) und dem Wellenbund (72) klemmt. Dieser Durchmesser ist größer als der Durchmesser der Kernwandung (84). Die Breite des Wellenbundes entspricht ca. der Breite des Dichtelements (85).

Für den Kolben (50) wird als Werkstoff ein Tetrafluorethylen/Hexafluorpropylen-Copolymer (FEP) verwendet. Dieser hat in Verbindung mit dem zuvor genannten Werkstoffen des Zylinders (10) selbstschmierende Eigenschaften, so dass zwischen dem Kolben (50) und dem Zylinder (10) keine separaten Schmiermittel benötigt werden. Als alternative Werkstoffe stehen u.a. Perfluoralkoxy-Copolymer (PFA), Ethylen-Tetrafluorethylen (E TFE) oder Polyvinylidenfluorid (PVDF) zur Auswahl. Eine hohe Schmierwirkung hat das z.B. nicht spritzgießbare Polytetraflourethylen (PTFE).

Ggf. kann auch eine Werkstoffkombination verwendet werden, bei der der Kernbereich (59, 61, 79) des Kolbens (50) aus einem Material geringerer Elastizität hergestellt wird, während die Schürzen (52, 72) aus einem hochelastischen Werkstoff gefertigt werden.

Die Dichtelemente (65, 85) sind zumindest nach den Figuren 1 bis 7 beispielsweise reine 0-Ringe, d.h. sie haben im unbelasteten Zustand jeweils einen kreisflächigen Einzelquerschnitt. In der jeweiligen Dichtstellung (67, 87) sind sie zwischen dem Kolben (50) und dem Zylinder (10) so positioniert, dass sie an den Wandungen beider Bauteile (10, 50) dicht anliegen. Hierbei werden sie jeweils elastisch verformt. Sie haben dann in der Regel eine von der Kreisfläche abweichende Querschnittsform.

Nach den Figuren 1 und 5 liegt der vordere O-Ring (65) im Zylinder (10) an der Zylinderinnenwandung (31) an. Den Kolben (50) berührt er an der Kernwandung (64), vgl. auch Figur 7, und an der Schürzeninnenkante (54). An den Wandungen (31) und (64) ist er aufgrund der dichtenden Einspannung elastisch abgeflacht. Der hintere O-Ring (85) sitzt eingeklemmt zwischen der Zylinderinnenwandung (31) und dem Wellenbund (72), vgl. Figur 7. Er ist hier z.B. stärker verformt als der vordere O-Ring.

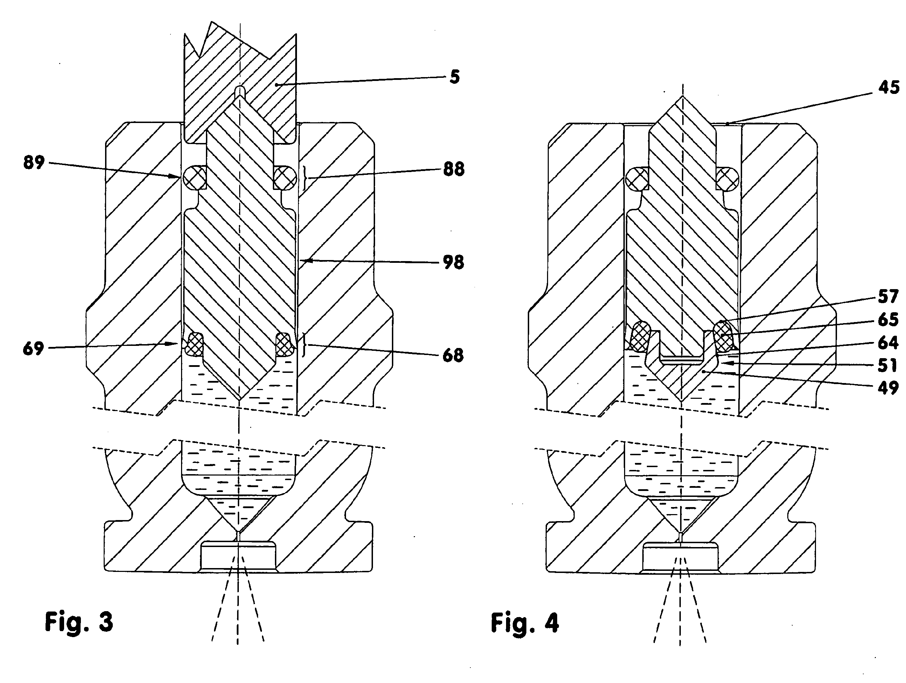

In Figur 3 ist der Kolben (50) in Bewegung dargestellt. Der vor dem Kolben (50) entstandene Staudruck hat den vorderen O-Ring (65) in die Axialringnut (57) gezwängt. Dabei hat der O-Ring (65) jeden Kontakt zur Zylinderinnenwandung (31) verloren. Dort liegt nur die Schürze (52) dichtend an. Der hintere O-Ring (85) ist unmittelbar nach dem Beginn der Kolbenbewegung durch die Reibung an der Zylinderinnenwandung (31) vom Wellenbund (72) gerutscht. Er liegt nun - mit geringer Restspannung auf der Kernwandung (84) auf. Der hintere O-Ring (85) hat ebenfalls keinen Kontakt zur Zylinderinnenwandung (31). Somit entfalten bei der Vorwärtsbewegung des Kolbens (50) die statischen Dichtelemente (65, 85) keine bremsende Wirkung.

In Figur 4 hat die vordere Axialringnut (57) - in der dargestellten Zeichnungsebene - eine andere Querschnittsform. Die Kernwandung (64) verjüngt sich von vorn nach hinten, so dass der O-Ring (65) beim Eindrücken in die Axialringnut (57) seinen mittleren Durchmesser verstärkt verkleinert. Der vordere Kolbenabschnitt (51) ist dazu als separate Kappe (49) gestaltet.

In den Figuren 5 und 6 sind Varianten zur Lagerung des hinteren O-Rings (85) dargestellt. In beiden Fällen liegt der O-Ring (85) an einem Gehäusebund (46) an. Nach der linksseitigen Variante ist der Durchmesser der radialen Wandung (47) so gewählt, dass der O-Ring (85) in seiner Dichtstellung (87) an der Wandung (61) des mittleren Kolbenabschnitts (91) anliegt. Bei der rechtsseitigen Variante liegt der O-Ring (85) halb im Zylinder- und halb im Kolbenbereich. In beiden Fällen wird der O-Ring beim Bewegungsbeginn des Kolbens (50) zwangsweise aus seiner Dichtstellung in seine Parkstellung geschoben, vgl. Figur 6. Er bleibt im Gehäusebund (46) liegen. Er kontaktiert dabei die Wandungen (47) und (48). Er berührt die Kernwandung (84) des Kolbens (50) nicht.

Die Figur 8 zeigt ein vorderes Dichtelement (105) in Form einer Lippendichtung. Die Lippendichtung (105) besteht beispielsweise aus einem rohrförmigen (106) und einem tellerförmigen Abschnitt (107). Der rohrförmige Abschnitt (106) liegt fest an der Kernwandung (84) an. Der tellerförmige Abschnitt (107) erstreckt sich von der Kernwandung (84) bis zur Zylinderinnenwandung (31). An der Zylinderinnenwandung (31) liegt er ebenfalls dicht an. Zusätzlich liegt er bei einem Kolben (50), der sich in der hinteren Kolbenposition (99) befindet, auf der Vorderkante der Schürze (52) auf. Wird der Kolben (50) vorwärts bewegt, drückt die von der Zylinder-Kolben-Einheit auszustoßende Flüssigkeit den tellerförmigen Abschnitt (107) unter bereichsweiser Verformung hinter die Schürze (52). Somit verliert die Außenkante des Abschnitts (107) den Kontakt zur Zylinderinnenwandung (31).

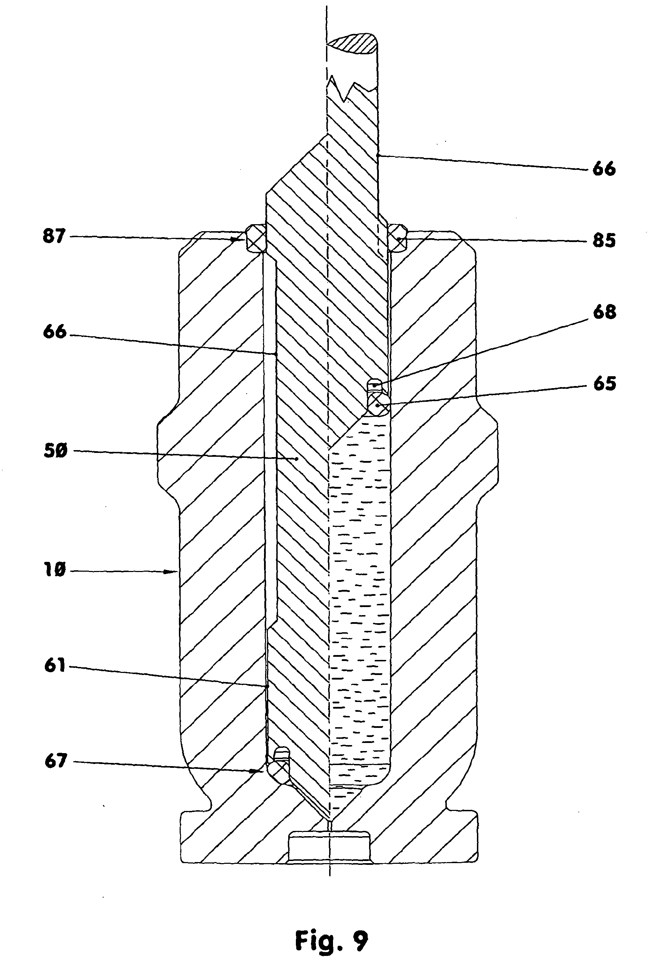

In Figur 9 wird ein Zylinder (10) gezeigt, in dem ein Kolben (50) geführt ist, dessen Dichtstellungen (67) und (87) weit auseinander liegen. Die Dichtstellungen (67) und (87) liegen jeweils in den vorderen und hinteren Endbereichen des Zylinders (10), vgl. linke Kolbenseite in Figur 9. Der mittlere Kolbenwandungsbereich (61) hat eine Taille (66), die sich bereichsweise zwischen den Dichtstellungen (67, 87) erstreckt. Der Durchmesser der Taille (66) ist so gewählt, dass das hintere Dichtelement (85), dessen Anlageverhältnisse in der Beschreibung zu Figur 5 erläutert sind, nur in den beiden in Figur 9 dargestellten Endlagen gleichzeitig am Kolben (50) und am Zylinder (10) anliegt.

Mit dieser Konstruktion wird auch ein eingefahrener Kolben (50), vgl. Figur 9, linke Kolbenseite, steril gegenüber dem Zylinder (10) abgedichtet. Eine derartige Zylinder-Kolben-Einheit kann vom Benutzer erst unmittelbar vor der Anwendung befüllt werden. Dazu wird der Zylinder (10) z.B. unter hydraulischem Ausdrücken des Kolbens (50) über die Freistrahlausnehmung (39) und die Düsenbohrung (36) befüllt. Beim hydraulischen Ausschieben des Kolbens (50), z.B. mit einer Befüllspritze, ist der Hydraulikdruck klein genug, um das Dichtelement (65) nicht in den Parkraum (68) zu pressen.

Alternativ kann das zu verabreichende Medikament auch in den Zylinder gesaugt werden. Dazu wird an dem der Düsenbohrung (36) abgewandten Ende des Kolbens (50) ein entsprechender Adapter angeordnet oder an- bzw. eingeformt. Über den - hier nicht dargestellten - Adapter lässt sich der Kolben (50) aus dem Zylinder (10) ziehen.

Hat der Kolben (50) im nun befüllten Zylinder (10) seine hintere Endlage erreicht, liegt das Dichtelement (85) an der Wandung (61) dichtend an. Ggf. kann die Taille (66) auch soweit nach unten verlängert sein, vgl. gestrichelte Linie in Figur 9, rechte Kolbenseite, dass das Dichtelement (85) den Kolben (50) in dieser Position zumindest nicht dichtend kontaktiert.

Die Zylinder-Kolben-Einheit nach Figur 9 kann somit sowohl vom Hersteller als auch vom Endverbraucher befüllt werden. In beiden Fällen ist der Innenraum der Zylinder-Kolbeneinheit, ob leer oder befüllt, im Bereich des Kolbens (50) steril verschlossen.

Die Dichtelemente (65, 85, 105) sind beispielsweise aus Silikon-, Chlor- oder Butylkautschuk hergestellt. Sie sind alle geschlossene Ringe. Ihre Einzelquerschnitte können jede beliebige Form haben. Ggf. weisen die Dichtungselemente (65, 85, 105) eines Kolbens (50) jeweils unterschiedliche Shore-Härten auf.

### Bezugszeichenliste:

- 1: Wirkstoff, Befüllung
- 5: Stempel für Kolbenantrieb
- 9: Mittellinie

- 10: Zylinder
- 11: vorderes Ende, Ende mit Austrittselement
- 12: Stirnfläche, vorn

- 15: hinteres Ende
- 16: Stirnfläche, hinten

- 20: Außenkontur
- 21: Adapterflansch

- 30: Kammer
- 31: Zylinderinnenwandung, Innenkontur
- 32: Zylinderboden
- 35: Ausströmtrichter
- 36: Düsenbohrung, Austrittselement
- 39: Freistrahlausnehmung

- 41: Öffnung, vorn
- 42: Fase
- 45: Öffnung, hinten
- 46: Gehäusebund
- 47: Bundfläche, radial
- 48: Bundfläche, axial
- 49: Kappe

- 50: Kolben
- 51: Kolbenabschnitt, vorn
- 52: Schürze, elastisch
- 53: Schürzenaußenkante, vorn
- 54: Schürzeninnenkante, vorn
- 55: Querschnitt zur Außenkante
- 56: Schürzeninnenwandung
- 57: Axialringnut
- 58: Kolbenkern, vorn
- 59: Kolbenspitze, vorn

- 61: Kolbenwandung, mittlere
- 62: Umrisslinie, gedacht
- 63: Querschnitt zur Umrisslinie (62)
- 64: Kernwandung, Kolbenwandung
- 65: Dichtelement, Kolbendichtung, O-Ring
- 66: Taille
- 67: Dichtstellung
- 68: Parkraum
- 69: Parkstellung

- 71: Kolbenabschnitt, hinten

- 72: Wellenbund

- 78: Kolbenkern, hinten
- 79: Kolbenspitze, hinten

- 84: Kernwandung
- 85: Dichtelement, Kolbendichtung, O-Ring
- 87: Dichtstellung
- 88: Parkraum
- 89: Parkstellung

- 91: Kolbenabschnitt, Mitte, Mittelabschnitt

- 98: Kolbenposition, zwischen den Endlagen
- 99: Kolbenposition, hinten, hintere Endlage

- 105: Dichtelement, Kolbendichtung, Lippendichtung, vorn
- 106: rohrförmiger Abschnitt
- 107: tellerförmiger Abschnitt

## Patentansprüche

1. Zylinderkolbeneinheit mit einem Zylinder (10) und einem darin geführten, steril gummiabgedichteten Kolben (50), wobei der Zylinder (10) und der Kolben (50) eine zumindest zeitweise wirkstoffbefüllbare Kammer (30) umschließen und der Zylinder (10) an seinem vorderen Ende (11) mindestens ein Austrittselement (36) aufweist, **dadurch gekennzeichnet,**
- **dass** der in einer hinteren Position (99) ruhende Kolben (50) gegenüber dem Zylinder (10) mit einem statischen vorderen (65) und mit einem statischen hinteren Dichtelement (85) steril abgedichtet ist, wobei beide Dichtelemente (65, 85) in einer Dichtstellung (67, 87) jeweils an der Wandung (31, 47) des Zylinders (10) und jeweils an der Wandung (64, 72) des Kolbens (50) anliegen,
- **dass** räumlich hinter jedem statischen Dichtelement (65, 85) ein, das jeweilige Dichtelement (64, 84) aufnehmender, Parkraum (68, 88) angeordnet ist,
- **dass** bei betätigtem Kolben (50) die einzelnen statischen Dichtelemente (65, 85) aus ihrer jeweiligen Dichtstellung (67, 87) heraus in eine im Parkraum (68, 88) gelegene Parkstellung (69, 89) überführt werden, wobei jedes Dichtelement (65, 85) in der Parkstellung (69, 89) entweder nur die Zylinderwandung (47, 48) oder nur die Kolbenwandung (64, 84) kontaktiert.
- **dass** zwischen den beiden statischen Dichtelementen (65, 85) mindestens ein dynamisches kolbenseitiges Dichtelement (52) angeordnet ist, das zumindest bei betätigtem Kolben (50) an der Zylinderinnenwandung (31) anliegt.

2. Zylinderkolbeneinheit gemäß Anspruch 1, **dadurch gekennzeichnet, dass** das dynamische Dichtelement (52) eine Dichtlippe in Form einer Schürze ist.

3. Zylinderkolbeneinheit gemäß Anspruch 2, **dadurch gekennzeichnet, dass** bei betätigtem Kolben (50) das vordere statische Dichtelement (65) an der Schürzeninnenwandung (56) anliegt.

4. Zylinderkolbeneinheit gemäß Anspruch 2, **dadurch gekennzeichnet, dass** die vordere Außenkante (53) der vorderen Schürze (52) bei betätigtem Kolben (50) eine Querschnittsfläche aufspannt, die der Querschnittsfläche der Zylinderinnenwandung (31) entspricht, die durch die Kontaktlinie der Außenkante (53) aufgespannt wird.

5. Zylinderkolbeneinheit gemäß Anspruch 2, **dadurch gekennzeichnet, dass** sich zwischen der Schürze (52) und dem Kolbenkern (58) eine sich axial erstreckende Ringnut (57) befindet, die der Parkraum (68) für die vordere statische Dichtung (65) ist.

6. Zylinderkolbeneinheit gemäß Anspruch 1, **dadurch gekennzeichnet, dass** bei dem in einer hinteren Position (99) ruhenden Kolben (50) das vordere statische Dichtelement (65) an der regulären Wandung (31) anliegt, während das hintere statische Dichtelement (85) einen Gehäusebund (46) kontaktiert, wobei der Gehäusebund (46) im Bereich der radialen Berührung durch das Dichtelement (85) einen mittleren Innendurchmesser hat, der größer ist als der mittlere Innendurchmesser der Zylinderinnenwandung (31).

## Claims

1. Cylinder/piston unit with a cylinder (10) and with a piston (50) which is guided therein and which is sealed off in a sterile manner by a rubber seal, the cylinder (10) and the piston (50) enclosing a chamber (30) that can be filled at least temporarily with active substance, and the cylinder (10) having at least one discharge element (36) at its front end (11), **characterized in that**
- the piston (50) resting in a rear position (99) is sealed off relative to the cylinder (10) in a sterile manner by a static front sealing element (65) and by a static rear sealing element (85), both sealing elements (65, 85) in a sealing position (67, 87) each bearing on the wall (31, 47) of the cylinder (10) and each on the wall (64, 72) of the piston (50),
- arranged spatially behind each static sealing element (65, 85), there is a parking area (68, 88) for receiving the respective sealing element (64, 84),
- when the piston (50) is actuated, the individual static sealing elements (65, 85) are transferred from their respective sealing position (67, 87) into a parked position (69, 89) located in the parking area (68, 88), and each sealing element (65, 85) in the parked position (69, 89) touches only the cylinder wall (47, 48) or only the piston wall (64, 84),
- at least one dynamic sealing element (52) on the piston side is arranged between the two static sealing elements (65, 85) and bears on the inner wall (31) of the cylinder at least when the piston (50) is actuated.

2. Cylinder/piston unit according to Claim 1, **characterized in that** the dynamic sealing element (52) is a sealing lip in the form of a skirt.

3. Cylinder/piston unit according to Claim 2, **characterized in that** the front static sealing element (65) bears on the inner wall (56) of the skirt when the piston (50) is actuated.

4. Cylinder/piston unit according to Claim 2, **characterized in that**, when the piston (50) is actuated, the front outer edge (53) of the front skirt (52) covers a cross-sectional surface area that corresponds to the cross-sectional surface area of the inner wall (31) of the cylinder covered by the contact line of the outer edge (53).

5. Cylinder/piston unit according to Claim 2, **characterized in that**, between the skirt (52) and the piston core (58), there is an axially extending annular groove (57), which is the parking area (68) for the front static seal (65).

6. cylinder/piston unit according to Claim 1, **characterized in that**, with the piston (50) resting in a rear position (99), the front static sealing element (65) bears on the regular wall (31), whereas the rear static sealing element (85) is in contact with a housing collar (46), which housing collar (46), in the area of radial contact by the sealing element (85), has a mean internal diameter that is greater than the mean internal diameter of the inner wall (31) of the cylinder.

## Revendications

1. Unité cylindre-piston comprenant un cylindre (10) et un piston (50) guidé dans celui-ci, pourvu d'une étanchéité en caoutchouc stérile, le cylindre (10) et le piston (50) renfermant une chambre (30) pouvant être remplie au moins temporairement avec une substance active, et le cylindre (10) présentant à son extrémité avant (11) au moins un élément de sortie (36),
**caractérisée en ce que**
- le piston (50) au repos dans une position arrière (99) est étanché de manière stérile par rapport au cylindre (10) avec un élément d'étanchéité statique avant (65) et un élément d'étanchéité statique arrière (85), les deux éléments d'étanchéité (65, 85) s'appliquant dans une position d'étanchéité (67, 87) à chaque fois contre la paroi (31, 47) du cylindre (10) et à chaque fois contre la paroi (64, 72) du piston (50),
- spatialement derrière chaque élément d'étanchéité statique (65, 85) est disposé un espace de positionnement (68, 88) recevant l'élément d'étanchéité associé (64, 84),
- lorsque le piston (50) est actionné, les éléments d'étanchéité statiques individuels (65, 85) sont transférés de leur position d'étanchéité respective (67, 87) dans une position de positionnement (69, 89) située dans l'espace de positionnement (68, 88), chaque élément d'étanchéité (65, 85) dans la position de positionnement (69, 89) venant en contact soit seulement avec la paroi du cylindre (47, 48), soit seulement avec la paroi du piston (64, 84),
- entre les deux éléments d'étanchéité statiques (65, 85) est disposé au moins un élément d'étanchéité dynamique (52) du côté du piston, qui s'applique contre la paroi interne du cylindre (31) au moins lorsque le piston (50) est activé.

2. Unité cylindre-piston selon la revendication 1, **caractérisée en ce que** l'élément d'étanchéité dynamique (52) est une lèvre d'étanchéité en forme de jupe.

3. Unité cylindre-piston selon la revendication 2, **caractérisée en ce que** lorsque le piston (50) est activé, l'élément d'étanchéité statique avant (65) s'applique contre la paroi intérieure de la jupe (56).

4. Unité cylindre-piston selon la revendication 2, **caractérisée en ce que** l'arête extérieure avant (53) de la jupe avant (52), lorsque le piston (50) est activé, décrit une surface en section transversale qui correspond à la surface en section transversale de la paroi intérieure du cylindre (31), qui est décrite par la ligne de contact de l'arête extérieure (53).

5. Unité cylindre-piston selon la revendication 2, **caractérisée en ce qu'**une rainure annulaire (57) s'étendant axialement se trouve entre la jupe (52) et le noyau du piston (58), laquelle rainure est l'espace de positionnement (68) pour le joint d'étanchéité statique avant (65).

6. Unité cylindre-piston selon la revendication 1, **caractérisée en ce que** lorsque le piston (50) repose dans une position arrière (99), l'élément d'étanchéité statique avant (65) s'applique contre la paroi régulière (31), tandis que l'élément d'étanchéité statique arrière (85) vient en contact avec un épaulement du boîtier (46), l'épaulement du boîtier (46) présentant dans la région du contact radial avec l'élément d'étanchéité (85), un diamètre intérieur moyen qui est supérieur au diamètre intérieur moyen de la paroi intérieure du cylindre (31).
